(19) Europäisches Patentamt
European Patent Office
Office européen des brevets

(11) EP 0 610 519 B1

(12) **EUROPEAN PATENT SPECIFICATION**

(45) Date of publication and mention of the grant of the patent: **01.12.1999 Bulletin 1999/48**

(21) Application number: **94908806.6**

(22) Date of filing: **20.08.1993**

(51) Int. Cl.$^6$: **A61K 31/34**, A61K 31/365, C07D 307/92, C07D 493/06, A61K 31/00

(86) International application number: **PCT/JP93/01167**

(87) International publication number: **WO 94/05283 (17.03.1994 Gazette 1994/07)**

# (54) ANTI-HIV DRUG

ANTI-HIV-MEDIKAMENT

MEDICAMENT ANTI-VIH

(84) Designated Contracting States: **DE FR GB IT**

(30) Priority: **02.09.1992 JP 23449292**

(43) Date of publication of application: **17.08.1994 Bulletin 1994/33**

(73) Proprietor:
**KYOWA HAKKO KOGYO CO., Ltd.**
**Chiyoda-ku, Tokyo 100 (JP)**

(72) Inventors:
- **YANO, Hiroshi**
  **Machida-shi, Tokyo 194 (JP)**
- **NAKANISHI, Satoshi**
  **Rockville, MD 20852 (US)**
- **MATSUDA, Yuzuru**
  **Koganei-shi, Tokyo 184 (JP)**
- **NONOMURA, Yoshiaki**
  **Suginami-ku, Tokyo 166 (JP)**
- **SASAKI, Hiroyuki**
  **Yokohama-shi, Kanagawa 247 (JP)**

(74) Representative:
**VOSSIUS & PARTNER**
**Postfach 86 07 67**
**81634 München (DE)**

(56) References cited:
**DE-A- 4 217 964** **JP-A- 3 215 423**

- **J. VIROL., vol. 64, no. 9, 1990, pages 4306-4312, XP000615986 KINTER ET AL.: "Direct and Cytokine-Mediated Activation of Protein Kinase C Induces Human Immunodeficiency Virus Expression in Chronically Infected Promonocytic Cells"**
- **BIOCHEM. BIOPHYS. RES. COMM., vol. 165, no. 3, 1989, P0G106, pages 1207-1212, XP002023786 TAKAHASHI ET AL.: "Hypericin and Pseudohypericin specifically Inhibit Protein Kinase C: Possible relation to their Antiviral Activity."**
- **J. BIOL. CHEM., vol. 267, no. 4, 1992, P0G106, pages 2157-2163, XP002023787 NAKANISHI: "Wortmannin, a Microbial Product Inhibitor of Myosin Light Chain."**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 610 519 B1

Printed by Xerox (UK) Business Services
2.16.7/3.6

## Description

Technical Field

The present invention relates to an anti-human immunodeficiency virus (HIV) agent containing, as an active ingredient, a myosin light chain kinase inhibitor such as wortmannin and MS-444. The anti-HIV agent of the present invention selectively inhibits the growth of HIV.

Background Art

HIV is the acquired immunodeficiency syndrome (AIDS)-causing pathogenic virus. Helper T-cells are infected with HIV, and the HIV proliferates in the infected helper T-cells and destroys the cells, which brings about the cellular immunodeficiency, resulting in the development of the symptoms of AIDS.

Azidothymidine (AZT) and interferon are known to be an anti-HIV agent. AZT has an action of selectively inhibiting reverse transcription in the life cycle of HIV comprising adhesion, invasion, uncoating, reverse transcription, integration, genetic transcription, protein synthesis, modification, assembly and budding. However, AZT has serious side effects, such as bone marrow suppression and dysfunction of the digestive and nervous system. Interferon is known to have an action of inhibiting budding of HIV.

Wortmannin represented by the following formula:

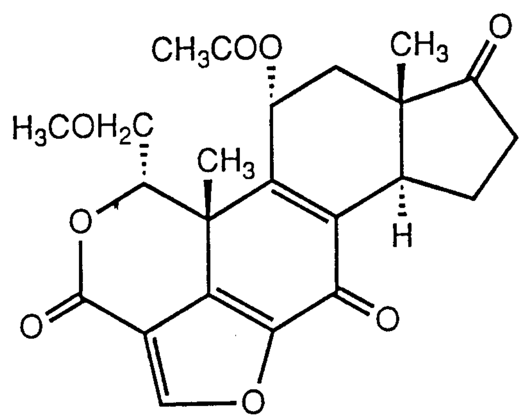

is known for its anti-inflammatory action [Experientia, 30, 135, (1974)] and vasodilator action [J. Biol. Chem., 267, 2157 (1992), JP-A-3-215423].

MS-444 represented by the following formula:

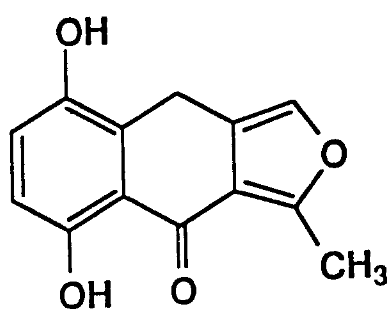

is known for its vasodilator action (WO93/09109).

Disclosure of the Invention

The present inventors have been interested in the "budding" phase in the life cycle of HIV for the purpose of selectively inhibiting metabolism of HIV without affecting metabolism of hosts. As a result of intensive studies, it has been found that a myosin light chain kinase inhibitor has an action of suppressing budding of HIV to thereby inhibit the

growth of HIV.

The present invention is directed to an anti-HIV agent containing a myosin light chain kinase inhibitor as an active ingredient.

The myosin light chain kinase inhibitor as used herein include wortmannin and MS-444.

Wortmannin is obtained by culturing a fungus of Penicillium wortmannii in a medium to allow the fungus to produce and accumulate wortmannin in the culture and recovering the wortmannin therefrom [Trans. Brit. Mycol. Soc., 40, 365 (1957), and J. Chem. Soc. Perkin I. 2898 (1972)].

MS-444 is obtained by culturing a fungus of the genus Micromonospora in a medium to allow the fungus to produce and accumulate MS-444 in the culture and recovering the MS-444 therefrom (WO93/09109).

Wortmannin and MS-444 were examined for the myosin light chain kinase inhibiting activity and the anti-HIV activity below.

Test Example 1

The myosin light chain kinase inhibitory activities of wortmannin and MS-444 were assayed according to the method of Nakanishi et al. [Analytical Biochemistry, 195, 313 (1991)]. Namely, 10 μl of methanol containing the test compound having various concentrations was added to 200 μl of a 25 mM Tris-HCl (pH 7.5) containing 0.5 mg/ml bovine serum albumin, 4.0 mM $MgCl_2$, 0.2 mM $CaCl_2$, 2.6 nM calmodulin, 24 μM peptide substrate (Lys-Lys-Arg-Pro-Gln-Arg-Ala-Thr-Ser-Asn-Val-Phe-Ser-$NH_2$), and 1.5 nM myosin light chain kinase, and the mixture was allowed to stand at 28°C for 10 minutes. The reaction was carried out by addition of 50 μl of 2 mM ATP. After 30 minutes, 100 μl of 10% acetic acid was added to the mixture to stop the reaction. The amounts of the phosphate-incorporated peptide substrate (peptide-P) and the nonphosphate peptide substrate (Peptide-non) were determined by HPLC. The inhibition rate was calculated according to the following equation.

$$\text{Rate of inhibition (\%)} = (1\text{-Ax\%/Ac\%}) \times 100$$

Ax%; 100 × (Peptide-P)/{(peptide-non) + (Peptide-P)} (in the presence of the test compound)
Ac%: 100 × (Peptide-P)/{(Peptide-non) + (Peptide-P)} (in the absence of the test compound)

The concentrations of the test compound inhibiting 50% of the myosin light chain kinase activity ($IC_{50}$) are shown in Table 1.

Table 1

| Test Compound | $IC_{50}$ (μM) |
|---|---|
| Wortmannin | 0.17 |
| MS-444 | 10 |

Test Example 2

Human lymphocyte-derived H-9 cells, infected with HIV (type $III_B$), were cultured in an RPMI-1640 medium (product of Biocell Laboratories) containing wortmannin and 10% fetal bovine serum (FBS) at 37°C for 15 minutes in a $CO_2$ incubator. The H-9 cells thus obtained were washed with an RPMI-1640 medium, and then cultured in another RPMI-1640 medium containing 10% FBS at 37°C for 360 minutes in a $CO_2$ incubator. The reverse transcriptase activity per unit culture medium was used as an index to determine the inhibitory effect of wortmannin on the budding of HIV.

The results are shown in Table 2.

Table 2

| Concentration of wortmannin (μM) | Budding of HIV (%) |
|---|---|
| 0.1 | 67 |
| 1.0 | 49 |
| 10 | 39 |

Host cell growth was not influenced by wortmannin in a concentration of 100 μM. The same test as mentioned above was repeated using MS-444 instead of wortmannin.

The results are shown in Table 3.

Table 3

| Concentration of MS-444 (μM) | Budding of HIV (%) |
|---|---|
| 10 | 95 |
| 100 | 82 |
| 1000 | 20 |

Host cell growth was not influenced by MS-444 in a concentration of 1000 μM.

The above results clearly show that the anti-HIV agent of the invention is expected to have excellent activity for inhibiting the growth of HIV without affecting host cells within therapeutically effective amounts.

The HIV agent of the present invention may be administered in the form of various pharmaceutical preparations either orally or parenterally. The pharmaceutical preparations may be in the dosage form of tablets, pills, powders, granules, capsules, suppositories, injections, or eye drops.

The above-described dosage forms can be formulated by the conventional methods. The preparation may contain excipients, lubricants, binders, disintegrators, suspending agents, isotonizing agents, emulsifiers and absorption accelerators.

The typical examples of the pharmaceutically acceptable carrier to be used in the present invention include water, distilled water for injection, physiological saline, glucose, fructose, sucrose, mannitol, lactose, starch, corn starch, potato starch, cellulose, methylcellulose, carboxymethylcellulose, hydroxypropylcellulose, alginic acid, talc, sodium citrate, calcium carbonate, calcium hydrophosphate, magnesium stearate, urea, silicone resin, sorbitan fatty acid ester, and glycerin fatty acid ester. Appropriate carriers are selected depending on the type of the dosage form.

The dosage of the myosin light chain kinase inhibitor for use for this purpose is determined depending on the desired therapeutic effect, the method of administration, the duration of administration, the age and the weight. The agent may be administered either orally or parenterally (for example, by injection, intraveous drip infusion, rectal administration using suppositories, and dermal application) at a daily dose of 0.01 to 2 mg/kg for a human adult.

The acute toxicity ($LD_{50}$) of wortmannin in the case of oral administration to rats has been reported to be 3 to 10 mg/kg [Experientia, 30, 135 (1974)].

With respect to the acute toxicity of MS-444, the mortality was observed in the case of oral administration to rats (300 mg/kg), or intraperitoneal administration to rats (100 mg/kg) and no rats died at such concentrations.

## Best Mode for Carrying Out the Invention

### Example 1 Tablets

| | |
|---|---|
| Wortmannin | 100g |
| Lactose | 40g |
| Corn starch | 18g |
| Carboxymethylcellulose calcium | 10g |

42 ml of a 10% hydroxypropylcellulose solution was added to the above components followed by mixing. The mixture was granulated using an extrusion granulator with a 1.0 mm basket attached. Fine granules for making tablets were produced by adding magnesium stearate. Tablets (170 mg), each having a diameter of 8 mm and containing 100 mg of wortmannin, were prepared by a conventional method.

Example 2 Capsules

| | |
|---|---|
| Wortmannin | 50g |
| Lactose | 80g |
| Potato starch | 38g |

42 ml of a 10% hydoxypropylcellulose solution was added to the above components followed by mixing. In the same manner as in Example 1, the mixture was granulated, and to the granules was added magnesium stearate. Capsules (170 mg) each containing 50 mg of wortmannin were prepared by a conventional method.

Example 3 Soft Capsules

10g of wortmannin was dissolved in 100g of soybean oil. The resulting solution was poured into the capsules by a conventional method to prepare soft capsules, each containing 10 mg of wortmannin.

Example 4 Tablets

| | |
|---|---|
| MS-444 | 200g |
| Lactose | 40g |
| Corn starch | 18g |
| Carboxymethylcellulose calcium | 10g |

42 ml of a 10% hydroxypropylcellulose solution was added to the above components followed by mixing. The mixture was granulated using an extrusion granulator with a 1.0 mm basket attached. Fine granules for making tablets were produced by adding magnesium stearate. Tablets (170 mg), each having a diameter of 8 mm and containing 200 mg of MS-444, were prepared by a conventional method.

Example 5 Capsules

| | |
|---|---|
| MS-444 | 100g |
| Lactose | 80g |
| Potato starch | 38g |

42 ml of a 10% hydroxypropylcellulose solution was added to the above components followed by mixing. In the same manner as in Example 4, the mixture was granulated, and to the granules was added magnesium stearate. Capsules (170 mg), each containing 100 mg of MS-444 were prepared by a conventional method.

Example 6 Soft Capsules

20g of MS-444 was dissolved in 100g of soybean oil. The resulting solution was poured into the capsules by

a conventional method to prepare soft capsules, each containing 20 mg of MS-444.

## Claims

1. Use of myosin light chain kinase inhibitor for the preparation of a pharmaceutical composition for use as an anti-human immunodeficiency virus (HIV) agent.

2. The use according to Claim 1, wherein the myosin light chain kinase inhibitor is selected from wortmannin and MS-444 represented by the following formula.

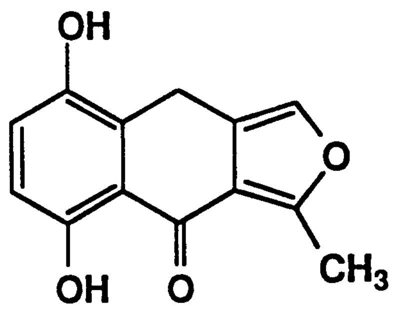

## Patentansprüche

1. Verwendung eines Inhibitors der Myosin-Leichtkettenkinase zur Herstellung eines Arzneimittels zur Verwendung als Mittel gegen das humane Immundefizienzvirus (HIV).

2. Verwendung nach Anspruch 1, wobei der Inhibitor der Myosin-Leichtkettenkinase ausgewählt ist aus Wortmannin und MS-444 der folgenden Formel:

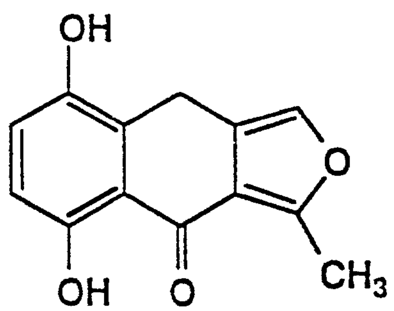

## Revendications

1. Emploi d'un inhibiteur de Kinase agissant sur les chaînes légères de myosine, pour préparer une composition pharmaceutique destinée à être utilisée comme agent anti-VIH (virus de l'immunodéficience humaine).

2. Emploi, conforme à la revendication 1, d'un inhibiteur de Kinase agissant sur les chaînes légères de myosine qui est choisi parmi la wortmannine et le composé MS-444 représenté par la formule suivante :